# EUROPEAN PATENT APPLICATION

(11) **EP 2 869 474 A1**
(43) Date of publication of application: **06.05.2015**
(21) Application number: 13306482.4
(22) Date of filing: 30.10.2013
(51) Int. Cl.: H04B 3/54, G06F 19/00

(54) **Medical device arrangement, adapter device and communication method**

(71) Applicant: Fresenius Vial SAS, 38590 Brézins (FR)
(72) Inventor: Doris, Lionel, 3800 Grenoble (FR)
(74) Representative: Fresenius Kabi Deutschland GmbH

(57) **Abstract**

The invention relates to a medical device arrangement, comprising a medical device (32); a communication interface (33) configured to allow the medical device (32) to communicate with other devices. According to the invention, the communication interface (33) is configured for establishing a communication link via a power line.

The invention also relates to an adapter device and a communication method.

## Description

The invention relates to a medical device arrangement according to the preamble of claim 1, an adapter device according to the preamble of claim 14 and a communication method according to the preamble of claim 15.

It is known to equip medical devices such as medical infusion pumps with communication capabilities in order to allow the medical devices to communicate with other devices (e.g. other medical devices). For example, wired technology (based e.g. on the Ethernet, USB, serial RS232 or Controller Area Network - CAN standard) or wireless technology (based e.g. on the Wi-Fi, Bluetooth or IRDA standard) is used.

It is an object of the invention to facilitate the communication with a medical device.

According to the invention a medical device arrangement is provided, comprising
- a medical device;
- a communication interface configured to allow the medical device to communicate with other devices,
- wherein the communication interface is configured for establishing a communication link via a power line.

The invention thus uses power line communication - PLC technology for communication with the medical device. In particular, the communication interface is configured for establishing a communication link via a DC power line such that an existing (e.g. low voltage) DC power line may also be used for communicating with the medical device (i.e. for transmitting and retrieving data to/from the medical device). For example, the DC power line at the same time is used for supplying electrical power to the medical device such that only a single line (that may consist of two wires) is required for both communication and power supply.

Data security and transmission is a very important aspect for medical devices and more particularly to the personal data of patients or the safety and reliability of operation of these devices. Unlike standard PLC networks that use the mains power grid on which the data can be easily intercepted or intentionally / unintentionally disturbed, the PLC network of the present invention (see below) in particular is designed to be physically defined, supplemented by screening measures and reduction of conducted and radiated electromagnetic noise to avoid any leaks and illegal interception of data. Data encryption may complement these security / privacy measures.

In particular, the DC power line comprises an electrical line (e.g. a power rail or a power bus) which, for example, extends in a (e.g. movable) rack installed, in particular, in a facility (such as a hospital) where the medical device is located. The rack may further be configured for receiving the medical device arrangement. For example, the medical device arrangement may be mounted to the rack. The DC power line may form an essentially closed DC power network, i.e. the DC power network has no direct connection to other communication networks and/or to other power networks. Therefore, using the DC power net a private (secure) communication network may be realized. Also, data communicated via the power line may be encrypted (using, for example, the 56 bits data encryption standard - DES). For example, all nodes (the medical devices connected to the DC power line) share a common encryption key.

Further, the communication via the DC power line may be based on the (e.g. multipoint) full-duplex or half-duplex method, wherein, broadband OFDM (orthogonal Frequency Division Multiplexing) multicarrier modulations in 1.6 to 30 MHz band may be employed. The principle of OFDM is to divide a large number of sub-carriers via which the digital signal is to be transmitted (as if the signal was transmitted over a large number of independent transmission systems at different frequencies).

Because the frequencies of the subcarriers are as close as possible and thus transmit the maximum amount of information on a given frequency portion, OFDM uses orthogonal subcarriers. The signals of different subcarriers overlap but due to the orthogonality do not interfere with each other. The signal to be transmitted is typically repeated on different subcarriers. Thus, in a transmission channel with multiple paths, where certain frequencies will be destroyed because of the destructive combination of paths, the system will still be able to recover the information using other carrier frequencies that have not been destroyed. Each subcarrier is independently modulated using digital modulations: For example, BPSK (Differential Binary Phase Shift Keying) or DQPSK ( Differential Quadrature Phase Shift Keying ).

It may be possible to transmit bitrates up to e.g. 14 Mbps. Further, data communication over the power line may include using quality of service control, in particular in order to guarantee a minimal or pre-determined bandwidth.

The medical device may be a medical infusion pump such as a syringe pump or a peristaltic pump. However, the invention is not restricted to infusion pumps. Rather, other medical devices such as medical monitoring devices may be equipped with a communication interface allowing communication via an existing DC power line.

According to another embodiment of the invention, the communication interface is integrated into the medical device, i.e. the medical device and the communication interface form a single unit. In particular, the communication interface and the medical device are arranged in a common housing.

The invention also relates to a communication network, comprising
- a plurality of medical device arrangements as described above;
- a DC power line,
- wherein the medical devices of the medical device arrangements are connected to the DC power line via the communication interfaces of the medical device arrangements in order establish a communication link between the medical devices and/or between the medical devices and at least one other device via the DC power line.

It is noted that the connection between the medical devices and the DC power line may be realized by electrically isolated coupling (e.g. capacitive or inductive coupling) such that there might be no galvanic connection between the communication interfaces and the DC power line.

The communication network may further comprise a communication gateway configured to manage a communication link between at least one of the medical devices connected to the DC power line (via their communication interfaces) and an external communication network and/or external devices connected to a communication line (different from the DC power line. In particular, the other communication network is not a power line communication network but a conventional wired communication network (e.g. an Ethernet, USB or serial network) or a conventional wireless communication network (a Wi-Fi network).

The communication gateway may provide physical and logical segregation (e.g.using separate hardware interfaces, RTOS - Real Time Operating System and microprocessor with MMU - Memory Management Unit) from different networks especially between "open" networks such as the hospital network and the "private" PLC communication network with medical devices. This separation is also true for the various vertical nodes - Columns - network communication PLC. The partitioning of networks and communication interfaces ensures an adequate level of reliability and security of the exchanged data.

Furthermore, the communication network may comprise a communication coupler for establishing an electrically isolated communication link between the DC power line and another communication line (belonging, for example, to an external communication network as set forth above). For example, the communication coupler is arranged between the above-mentioned communication gateway and the DC power line such that a (non-galvanic) communication link between the communication gateway and the DC power line is established by means of the communication coupler.

The communication coupler may use capacitive or inductive coupling between the DC power line and the other communication line.

According to another embodiment of the invention, the communication network further comprises a medical device control unit for controlling at least one of the medical devices via the DC power line, i.e. via a communication link established between the medical control unit and the medical devices via the DC power line. The medical device control unit may supervise at least some of the medical devices. For example, the medical device control is used for setting parameters of the medical devices and/or for reading data from the medical devices.

Moreover, the medical device control unit may be connected to the DC power line via the above-mentioned communication gateway, i.e. the medical device control unit is not directly connected to the DC power line.

The communication network according to the invention may further comprise a power supply unit for supplying DC power to the medical devices via the DC power line. In particular, the power supply unit is configured for converting AC power (provided by an external AC power source such as a central power source of a facility) into DC power supplied to the medical devices via the DC power line.

In particular, the power supply unit is capable of supplying a low DC voltage (e.g. 12 Volts) to the medical devices connected to the DC power line.

The communication network according to the invention may further comprise a filter unit arranged between the power supply unit (in particular, arranged close to the power supply unit) and the medical device arrangements, the filter unit being configured for filtering voltages generated by the power supply unit having frequencies that may disturb the communication via the DC power line, e.g. frequencies that are used for the communication via the DC power line.

Further, the filter unit may also be configured to prevent communication signals transmitted over the DC power line from leaving the DC power line via the power supply unit. That is, the filter unit is configured for securing the privacy of the DC power line communication network and also may protect an external power network connected to the power supply unit from electric disturbances.

Adapter device for establishing a communication link between a medical device arrangement as described above and another device, comprising
- a power line for supplying electrical power to the medical device arrangement;
- a connector for connecting the communication interface of the medical device arrangement to the power line; and
- a communication coupler for establishing an electrically isolated communication link between the power line and the other device.

The adapter device may be a portable stand-alone unit used in particular for connecting a single medical device arrangement to the other device (e.g. a local host computer) as described in more detail below.

The invention also relates to a communication method comprising providing a plurality of medical devices and establishing a communication link between the medical devices and/or between the medical devices and at least one other device via a DC power line. In particular, a plurality of medical device arrangements (each comprising the described medical device and the described communication interface) as described above are employed for establishing the communication link.

Embodiments of the invention are described in more detail hereinafter with reference to the Figures, wherein
- Fig. 1: shows a block diagram of a communication network according to a first embodiment of the invention;
- Fig. 2: shows a block diagram of a communication network according to a second embodiment of the invention; and
- Fig. 3: depicts an adapter device according to an embodiment of the invention.

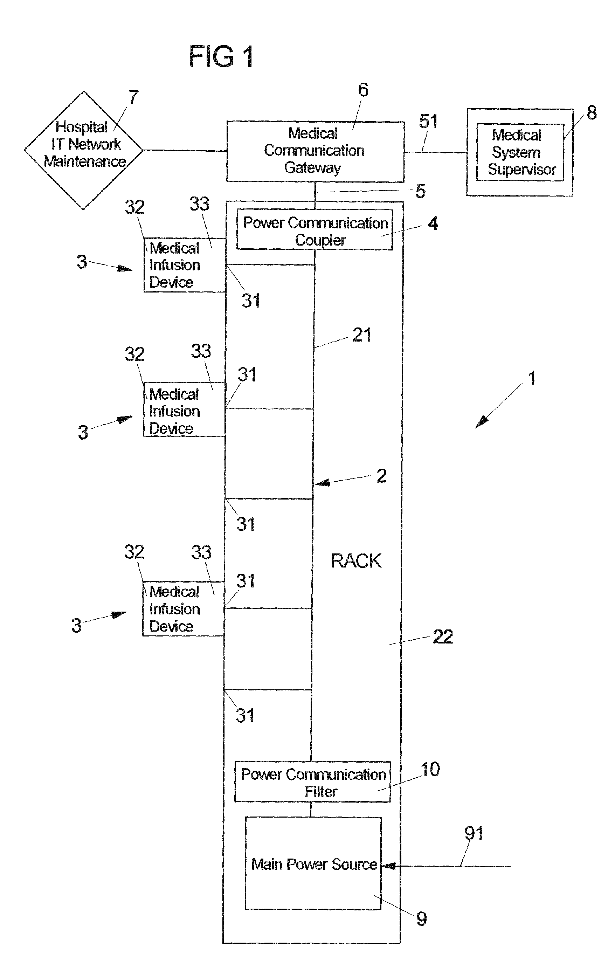

More particularly, Figure 1 depicts a communication network in the form of a power line communication (PLC) network 1. The PLC network 1 comprises a DC power line 2 for supplying DC power to a plurality of medical device arrangements in the form of medical infusion device arrangements 3. The DC power line 2 comprises a power rail 21 arranged in a (e.g. modular) rack 22, the rack 22 providing mechanical support for power rail 21. The power rail 21 is connected to power interfaces 31 (e.g. in the form of power plugs) via which DC power conveyed via the power rail 21 is supplied to the medical infusion device arrangements 3. According to the Figure, not all the of the power interfaces 31 are used. Of course, more or less power interfaces 31 can be provided.

The medical infusion device arrangements 3 each comprise a medical device in the form of a medical infusion device 32 and a communication interface 33 (e.g. comprising at least one hardware card) via which a data communication link between the medical infusion devices 32 or between the medical devices 32 and an external device (not shown) via the DC power line 2 can be established. For this, the medical infusion devices 32 are connected to the DC power line 2 via the communication interfaces 33, which, in turn, may be connected to the power interfaces 31 (using e.g. isolated coupling such a capacitive or inductive coupling). However, it is also possible that besides the power interfaces 31 additional plugs are used for connecting the communication interfaces 33 to the DC power line 2. Thus, the DC power line 2 is not only used for supplying power to the medical infusion devices 32, but also as a communication backbone for the medical infusion devices 32. Due to the essentially closed architecture of the DC power line 2, the PLC network 1 realized by the DC power line 2 is a secure (private) network.

The communication network 1 further comprises a communication coupler in the form of a power communication coupler 4. The power communication coupler 4 provides an electrically isolated communication link between the DC power line 2 and an external communication line 5 (separate from the DC power line 2), which, in turn, connects to an external communication network 7 and/or an external medical device control unit in the form of a medical system supervisor 8. For example, the power communication coupler 4 uses capacitive or inductive coupling for establishing the communication link between the DC power line 2 and the external line 5, wherein the DC power line 2 (and the rack 22) are electrically isolated from the external communication line 5. Further, the power communication coupler 4 blocks the DC component of the voltages on the DC power line 2 but let pass (composite) frequencies used for data communication via the DC power line 2 towards the external communication network 7 and/or the medical system supervisor 8.

The PLC network 1 also comprises a communication gateway 6 (e.g. in the form of a communication hub or a communication switch) for managing the communication between the medical infusion devices 3 connected to the DC power line 2 and the external communication network 7 (different from the PLC network) and/or the medical system supervisor 8. The external communication network 7 may be a Wi-Fi network or a wired network such as Ethernet, USB or a serial network. For example, the external network 7 is a main communication network of a facility (such as a hospital) where the medical infusion device arrangements 3 are located.

The medical system supervisor 8 is connected to the medical communication gateway 6 via another communication line 51. Further, the medical system supervisor 8 is configured for controlling the medical infusion devices 32, wherein the medical system supervisor 8 communicates with the medical infusion devices 32 via the DC power line 2. For example, the medical system supervisor 8 is able to set parameters or read data from the medical infusion devices 32 via the DC power line 2. It is noted that in addition or instead of the medical system supervisor 8 other external devices (e.g. other medical devices) may be connected to the DC power line 2 via the medical communication gateway 6 and the power communication coupler 4.

The PLC network 1 further comprises a power supply unit in the form of a main power source 9 for supplying DC power (in particular low voltage DC power) to the medical infusion devices 32 via the DC power line 2. For this, the main power source 9 is connected to an external power source (not shown) via an external power line 91 or power network, wherein the external (e.g. public) power source may be an AC power source. The main power source 9 connected to the DC power line 2 may be arranged in rack 22.

Arranged close to the main power source 9 is a filter unit in the form of power communication filter 10 used for filtering voltages generated by the main power source 9 that have frequencies which may disturb the data communication over the DC power line 2. The power communication filter 10 may also block frequencies used for data communication over the DC power line 2 to prevent these frequencies from leaving the DC power line 2 (i.e. the PLC network 1) via the main power source 9 such that disturbances of the facility's main power net and/or leakage of data signals communicated via the PLC network 1 via the main power source 9 (e.g. by conductive coupling to the external power line 91) are thwarted. Thus, power communication filter 10 is also used for securing the privacy of the PLC network 1.

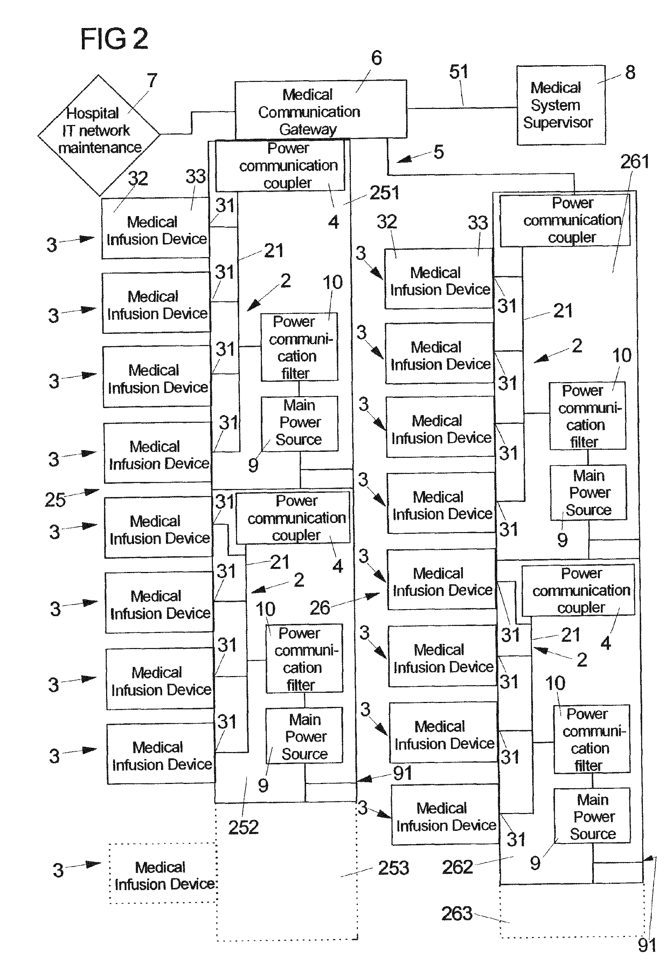

Figure 2 depicts a modification of the embodiment shown in Figure 1. According to Figure 2, a plurality of medical infusion device arrangements 3 (including medical infusion devices 32 and communication interfaces 33) is arranged in two rack columns 25, 26. Each one of the two rack columns 25, 26 supports, for example, three racks 251-253, 261-263 that each may receive four medical infusion device arrangements 3 such that by using two rack columns a total of 24 medical infusion device arrangements 3 (i.e. medical infusion devices 32) may be arranged. The medical infusion device arrangements 3 arranged in rack column 25 and those arranged in rack column 26 each are connected to a rack internal DC power lines 2 via their communication interfaces 33 similar to Figure 1. The DC power lines 2 of the racks of one rack column 25, 26 are connected to one another.

Further, one of the rack columns (rack column 25) is directly connected to the medical communication gateway 6, while the other rack column 26 is connected to the medical communication gateway 6 by means of an external line 5 (e.g. a cable). Also, each one of the racks 251-253, 261-263 may comprise a power communication coupler 4, a power communication filter 10 and a main power source (see description of Figure 1). However, only one of the racks (e.g. the bottom one) of each rack column 25, 26 may be connected to the external power source (not shown) via line 91, wherein the power will be transmitted internally to the other racks via the interconnected power lines 2. Similarly, the rack columns 25, 26 may be connected to the power communication gateway 6 via the power communication coupler 4 of only one of the racks of the columns 25, 26.

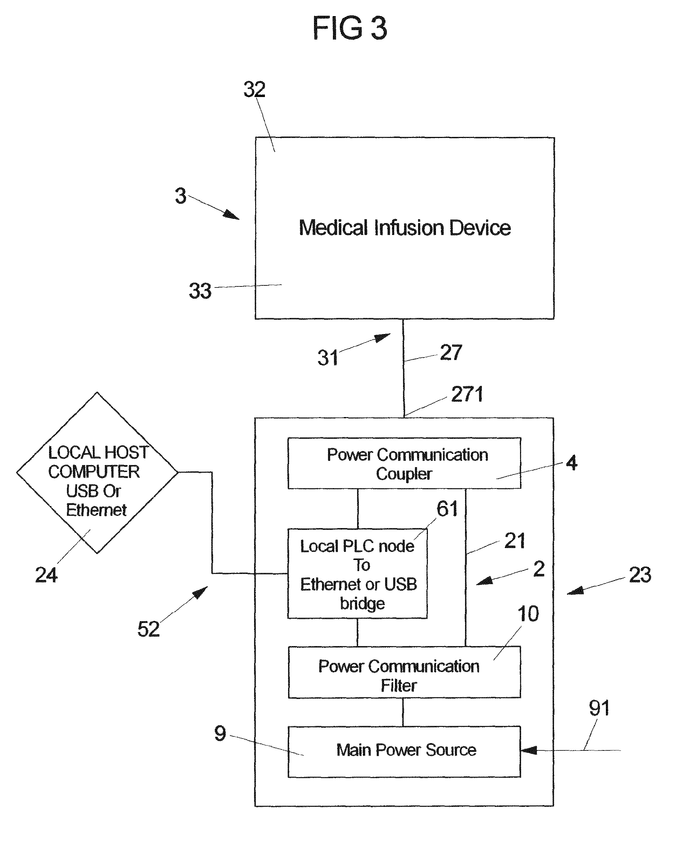

Figure 3 shows an adapter device for establishing a communication link between a single medical device arrangement 3 and another device (e.g. a local host computer 24). The adapter device is an adapter rack 23 configured similarly to the racks 22, 251-253 and 261-263 discussed above. That is, rack 23 comprises a DC power line 2 (including power rail 21) via which DC power generated by the main power source 9 (connected to external power line 91) is supplied to medical infusion device arrangement 3 (which is connected to the rack 23 via a line 27). The adapter rack 23 may be a (e.g. portable) stand-alone unit.

Further, the communication interface 33 of the medical infusion device arrangement 3 is connected to the DC power line 2 of adapter rack 23 via a connector 271 (and e.g. also via line 27) such that a communication link to the medical infusion device 32 of the medical infusion device arrangement 3 via the DC power line 2 can be realized. Adapter rack 23 further comprises a power communication coupler 4 providing an electrically isolated communication link between the DC power line 2 and a communication line 52 (such as a USB or Ethernet line) via which the local host computer 24 is connected to the adapter rack 23. Moreover, the adapter rack 23 comprises a communication bridge 61 arranged between the power communication coupler 4 and the communication line 52 (or a connector to which the communication line 52 is connected). In particular, the communication bridge 61 is configured to transform a custom specific protocol used for the communication over the DC power line to a standard protocol such as USB or Ethernet.

The adapter rack 23 also comprises a power communication filter 10 for securing privacy of the power line communication as set forth above. The main power source 9 may be dimensioned smaller than the power sources shown in Fig. 1 and 2. For example, power source 9 of Fig. 3 is configured to supply power to a single medical device arrangement, only.

### Reference signs

- 1: PLC network
- 2: DC power line
- 3: medical infusion device arrangement
- 4: power communication coupler
- 5: external line
- 6: medical communication gateway
- 7: external communication network
- 8: medical system supervisor
- 9: main power source
- 10: power communication filter
- 21: power rail
- 22, 251-253, 261-263: rack
- 23: adapter rack
- 24: local host computer
- 25, 26: rack column
- 27: line
- 31: power interfaces
- 32: medical infusion device
- 33: communication interface
- 51, 52: communication line
- 61: communication bridge

## Claims

1. A medical device arrangement, comprising
- a medical device (32);
- a communication interface (33) configured to allow the medical device (32) to communicate with other devices;
**characterized in that**
the communication interface (33) is configured for establishing a communication link via a power line.

2. The medical device as claimed in claim 1, wherein the communication interface (33) is configured for establishing a communication link via a DC power line (2).

3. The medical device as claimed in claim 1 or 2, wherein the medical device (32) is an infusion pump.

4. A communication network, comprising
- a plurality of medical device arrangements (3) as claimed in one of the preceding claims;
- a DC power line (2),
- wherein the medical devices (32) of the medical device arrangements (3) are connected to the DC power line (2) via the communication interfaces (33) of the medical device arrangements (3) in order establish a communication link between the medical devices (32) and/or between the medical devices (32) and at least one other device via the DC power line (2).

5. The communication network as claimed in claim 4, wherein also electric power is supplied to the medical devices (32) via the DC power line (2).

6. The communication network as claimed in claim 4 or 5, further comprising a communication gateway (6) configured to manage a communication link between at least one of the medical devices (32) connected to the DC power line (2) and an external communication network (7) and/or at least one external device connected to a communication line (5, 51) different from the DC power line (2).

7. The communication network as claimed in one of the claims 4 to 6, further comprising a communication coupler (4) for establishing an electrically isolated communication link between the DC power line (2) and an external communication network (7) and/or an external device connected to a communication line (5, 51) different from the DC power line (2).

8. The communication network as claimed in claim 6 and 7, wherein a communication link between the communication gateway (6) and the DC power line (2) is established by means of the communication coupler (4).

9. The communication network as claimed in claim 7 or 8, wherein the communication coupler (4) uses capacitive or inductive coupling.

10. The communication network as claimed in one of the claims 4 to 9, further comprising a medical device control unit (8) for controlling at least one of the medical devices (32) via the DC power line (2).

11. The communication network as claimed in claim 10 as far as dependent on claim 6, wherein the medical device control unit (8) is connected to the DC power line (2) via the communication gateway (6).

12. The communication network as claimed in one of the claims 4 to 11, further comprising a power supply unit (9) for supplying DC power to the medical devices (32) via the DC power line (2).

13. The communication network as claimed in claim 12, further comprising a filter unit (10) arranged between the power supply unit (9) and the medical device arrangements (3) for filtering voltages generated by the power supply unit (9) having frequencies that may disturb the communication over the DC power line (2).

14. Adapter device for establishing a communication link between a medical device arrangement as claimed in one of the preceding claims and another device, comprising
- a power line for supplying electrical power to the medical device arrangement (3);
- a connector (271) for connecting the communication interface (33) of the medical device arrangement (3) to the power line
**characterized by**
a communication coupler (4) for establishing an electrically isolated communication link between the power line and the other device (24).

15. Communication method comprising providing a plurality of medical devices, **characterized by** establishing a communication link between the medical devices (32) and/or between the medical devices (32) and at least one other device via a DC power line (2).
